# EUROPÄISCHE PATENTSCHRIFT

(11) **EP 0 754 755 B1**
(45) Veröffentlichungstag und Bekanntmachung des Hinweises auf die Patenterteilung: **17.12.2003**
(21) Anmeldenummer: 96111173.9
(22) Anmeldetag: 11.07.1996
(51) Int. Cl.: C12N 15/38, C12N 15/62, G01N 33/569, C07K 14/05

(54) **Rekombinante autologe Fusionsproteine des Epstein-Barr-Virus, diese enthaltende Testkits und Verfahren zum Nachweis von Epstein-Barr-Virus-spezifischen Antikörpern**
Recombinant autologous Epstein-Barr viral fusion proteins, test kits containing them and methods for the detection of Epstein-Barr virus specific antibodies
Protéines, fusionnées recombinantes autologues du virus d'Epstein-Barr, trousse d'essais les contenant ainsi que procédé pour la détection d'anticorps spécifiques du virus d'Epstein-Barr

(30) Priorität: 19.07.1995 DE 19526384
(43) Veröffentlichungstag der Anmeldung: 22.01.1997
(73) Patentinhaber: BIOTEST AG, D-63303 Dreieich (DE)
(72) Erfinder: Hinderer, Walter, Dr., 63110 Rodgau (DE); Vornhagen, Rolf, Dr., 63225 Langen (DE); Lang, Dieter, Dr., 63322 Rödermark (DE); Sonneborn, Hans-H., Dr., 63150 Heusenstamm (DE)
(74) Vertreter: Keller, Günter, Dr.

(56) Entgegenhaltungen:
- EP-A- 0 173 254
- EP-A- 0 281 803
- EP-A- 0 574 048
- WO-A-95/03415

## Beschreibung

Die vorliegende Erfindung betrifft Fusionsproteine, die immunologisch mit Antikörpern gegen das Epstein-Barr-Virus (EBV) reagieren, Testkits, die diese Fusionsproteine enthalten und Verfahren zum Nachweis von Antikörpern gegen Epstein-Barr-Virus.

Das Epstein-Barr-Virus (im folgenden kurz: EBV) ist ein weit verbreitetes menschliches Herpesvirus, das in Zusammenhang mit dem sogenannten Burkitt Lymphom entdecket wurde. Das Burkitt Lymphom hat seine Hauptverbreitung in Zentral-Afrika und Neuguinea und tritt dort überwiegend bei Kindern auf.

Es wurde auch herausgefunden, daß das Virus bei dem nasopharyngealen Karzinom auftritt. Das nasopharyngeale Karzinom entsteht durch Transformation von Epithelzellen im Nasopharynx. Neben EBV begünstigen wahrscheinlich auch karzinogene Substanzen in Nahrungsmitteln und genetische Faktoren die Entstehung von nasopharyngialem Karzinom. Im fortgeschrittenen Stadium treten häufig Metastasen in Lymphknoten auf. Süd-China ist ein Hochrisiko-Gebiet für diese Erkrankung.

Es konnte auch gezeigt werden, daß das EBV der Verursacher der "mononucleosis infectiosa" ist. Die infektiöse Mononukleose wird auch als Pfeiffer'sches Drüsenfieber (morbus Pfeiffer) bezeichnet. Das Krankheitsbild der infektiösen Mononukleose zeigt sich als Folge einer EBV-Primärinfektion. Betroffen sind vor allem Kinder und junge Erwachsene. Bei Kindern verläuft die Krankheit häufig asymptomatisch. Die akute Krankheit kann folgende Symptome zeigen: Fieber, Pharyngitis, Tonsilitis, Lymphadenopathie, Kopf- und Muskelschmerzen, Müdigkeit, Leukozytose, Ausschlag, Splenomegalie und Hepatitis. Beweisend für die mononucleosis infectiosa ist - unter Einschluß des IgM-Titers - der Nachweis von Antikörpern gegen das Epstein-Barr-Virus.

Soweit bisher bekannt ist, sind alle Menschen, die einmal mit EBV infiziert wurden, lebenslang latent infiziert. Dies äußert sich durch die lebenslange Gegenwart einer kleinen Anzahl von mit dem EBV-Genom transformierten B-Zellen unter den zirkulierenden peripheren Blutlymphozyten. Daher können bei derartigen Patienten permanent IgG-Antikörper gegen EBV nachgewiesen werden. Da ein relativ großer Anteil latent inf izierter Personen transient oder permanent freies EBV im Speichel oder über den Genitaltrakt ausscheidet, was für die Verbreitung des EBV essentiell ist, kann eine Persistenz von EBV auch in Epithelzellen angenommen werden. Die Epithelzellen werden, so wird angenommen, in frühen Differenzierungsstadien aus dem Reservoir EBV-positiver lymphoider Zellen laufend infiziert. Die Übertragung von EBV erfolgt in der Regel nicht durch Aerosol oder flüchtigen Kontakt, sondern setzt einen engen Schleimhautkontakt voraus ("kissing disease").

Als Folge einer Immun-Defizienz oder Immun-Suppression, die beispielsweise bei AIDS-Patienten oder bei Transplantationspatienten mit immunsuppressiver Therapie auftreten kann, können sich das EBV-Genom tragende lymphoide Zellen unkontrolliert (poly-/oligoklonal) vermehren und Lymphome bilden. Bei einer länger andauernden Schwächung des zellulären Immunsystems, z.B. auch bei hochdosierter zytostatischer Therapie, kann es zu monoklonalen B-Zell-Lymphomen kommen.

Bei der Kontrolle der EBV-Infektion im Körper spielt das körpereigene Immunsystem eine wesentliche Rolle. Für die Diagnostik und Therapie von verschiedenen Erkrankungen und insbesondere bei Patienten, deren Immunsystem geschwächt ist, ist es daher wesentlich, die Frage zu beantworten, ob es sich um eine Primärinfektion von EBV handelt, oder ob eine Aktivierung des bereits im Körper vorhandenen Virus vorliegt.

Die Diagnostik von EBV-Infektionen erfolgt entweder direkt im betroffenen Gewebe durch Nachweis viraler Nukleinsäuren oder durch den Nachweis von EBV-kodierten Proteinen mit Hilfe monoklonaler Antikörper oder indirekt, d.h. es werden Antikörper gegen EBV-Proteine bestimmt.

Für eine differenzierte EBV-Serodiagnostik wurde bisher häufig die Immunfluoreszenz (IF-Diagnostik) verwendet. Dabei werden die jeweiligen Antikörper gegen verschiedene EBV-Antigenkomplexe dadurch nachgewiesen, daß Zellinien, die bestimmte EBV-Antigene exprimieren, auf Objektträgern fixiert werden. Diese werden in Kontakt mit verdünnten Patientenseren gebracht und die Bindung von Antikörpern an die EBV-spezifischen Antigene wird mit Hilfe der indirekten Immunfluoreszenz nachgewiesen.

Aufgrund der Immunfluoreszenzdiagnostik entstand historisch die folgende Einteilung der EBV-Antigene:

### a) Epstein-Barr Nukleäre Antigene (EBNA):

EBNA-1-6; diese Proteine sind in latent infizierten Zellen ausgeprägt. Neben EBNA sind noch weitere EBV-Proteine wie latente Membranproteine, LMP1 bzw. LMP2 nachweisbar.

### b) Frühantigene (early antigens, EA):

Hierbei handelt es sich um frühe Proteine der Virusreplikation. Unterscheidbar sind sie aufgrund des unterschiedlichen Fixierungsverhaltens. Sie werden bezeichnet als EA-D (diffuse) und EA-R (restricted).

### c) Viruskapsid Antigene (VCA):

Hierbei handelt es sich um Strukturproteine des Viruspartikels aus der späten Phase (lytisches Stadium).

Diese Antigenklassen waren ursprünglich rein serologische Definitionen. Jeder Komplex setzt sich aus vielen verschiedenen EBV-kodierten Polypeptiden zusammen. Eine vollständige Definition auf Proteinebene, mit Ausnahme der EBNAs, liegt bisher nicht vor. Bis heute sind die Immunfluoreszenz-Tests eine weit verbreitete Methode zur Bestätigung akuter EBV-Infektionen. Die wichtigsten Seromarker sind dabei VCA-IgM zur Diagnose von Primärinfektionen und VCA-IgG als Beweis der stattgehabten Infektion (epidemiologischer Marker). Zusätzlich sind VCA-IgA-Antikörper ein wichtiger Seromarker für die Diagnose und Prognose von dem nasopharyngialen Karzinom, insbesondere in Risikogebieten wie China. Antikörper gegen die Frühantigene, vor allem EA-D-IgG, werden zur Definition von Reaktivierungen eingesetzt.

Obwohl die Immunfluoreszenz weitverbreitet ist und als "Goldstandard" akzeptiert wird, hat sie eine Reihe von Nachteilen. Die Tests sind zeitaufwendig und laborintensiv und die Durchführung und Auswertung ist abhängig von erfahrenem Personal. Eine Automatisierung ist nicht möglich, d.h. die Immunfluoreszenz-Tests sind nicht geeignet für große Probenaufkommen. Quantitative Aussagen sind nur über Verdünnungsreihen möglich, die aber aufgrund der Variabilität der Antigenmenge in den produzierenden Zellinien, die auf Objektträger aufgebracht werden und dem subjektiven Ablesen nur schwer standardisiert werden können. Außerdem sind die Tests störanfällig gegenüber Autoantikörpern, die mit zellulären Proteinen reagieren. Hierbei kann es sich um antinukleäre, anti-mitochondriale und anti-DNA Antikörper handeln. Dies ist ein generelles Problem von Tests, die komplette Zellen oder nicht-gereinigte Antigene verwenden.

Ausgehend von den oben beschriebenen Schwierigkeiten wurde versucht, die EBV-Diagnose zu vereinfachen und zu verbessern. Häufig werden hierbei Mikrotiterplatten verwendet, die mit isolierten Antigenen beschichtet werden (insbesondere ELISA-Tests). Bei den EBV-ELISAs wurden zunächst natürliche Antigene verwendet, die aus Zellkulturen isoliert wurden. Hierzu wurde das Antigenmaterial entweder als angereichertes Vollantigen (Viruspellets) eingesetzt oder in Form von mit Hilfe monoklonaler Antikörper immunaffinitätsgereinigte Einzelproteine verwendet. Für derartige Tests muß aber bekannt sein, welche Antigene diagnostisch geeignet sind im Hinblick auf Spezifität und Sensitivität. Ein Problem besteht auch darin, daß innerhalb der Herpesviren zum Teil große Sequenzhomologien auftreten. Deshalb müssen solche Antigene ausgewählt werden, die einerseits reaktiv sind, aber andererseits nicht kreuzreaktiv sein dürfen. Bei der Verwendung von natürlichen Antigenen stellt sich das Problem, daß die Bereitstellung der Antigene in wirtschaftlicher Weise und in stets gleichbleibender Qualität nicht immer leicht zu bewerkstelligen ist. Daher können in den Tests rekombinante Antigene oder Peptide verwendet werden oder Antigene können mit chemischen Methoden synthetisiert werden. Bei der chemischen Synthese von Peptiden tritt allerdings die Schwierigkeit auf, daß lange Polypeptide nur mit sehr geringer Ausbeute hergestellt werden können. Bei der chemischen Synthese von Peptiden stellt sich auch häufig das weitere Problem, daß Konformationsepitope nicht korrekt dargestellt werden können. Durch die Beschränkung auf nur wenige Epitope bei Verwendung von z.B. synthetischen Oligopeptiden läuft man prinzipiell Gefahr, nicht alle Varianten, d.h. Subtypen von EBV serologisch zu erfassen. Gerade EBV ist bekannt für eine große Sequenzvariabilität. Bei der gentechnologischen Herstellung von viralen Polypeptiden stellt sich häufig das Problem, daß eine ausreichende Expression der gewünschten Polypeptide nur schwierig zu bewerkstelligen ist.

Bei der Verwendung prokaryotischer Expressionssysteme, wie z.B. E.coli, stellt sich zusätzlich das Problem, daß die rekombinanten Antigene hochgereinigt werden müssen, da noch verbleibende Kontaminationen prinzipiell im Menschen immunogen sind, d.h. serologisch zu falsch-positiven Resultaten führen können.

Aufgabe der vorliegenden Erfindung ist daher die Bereitstellung von diagnostisch relevanten Antigenen zum Nachweis von EBV-Infektionen.

Gegenstand der vorliegenden Erfindung sind daher autologe Fusionsproteine, die dadurch gekennzeichnet sind, daß sie
- eine erste Aminosäuresequenz aufweisen, die wenigstens den ersten 100 Aminosäuren, einschließlich des N-Terminus, des p23-Proteins des Epstein-Barr-Virus entspricht und wenigstens
- eine weitere Aminosäuresequenz aufweisen, die der Aminosäuresequenz des C-terminalen Bereichs des Epstein-Barr-Virusproteins mit der Bezeichnung p18 oder eines Teils dieses Bereichs entspricht und die immunologischen Eigenschaften eines serologisch (IF) als "Viruskapsid Antigen" definierten späten Antigens aufweist.

Bevorzugt handelt es sich bei der ersten Aminosäuresequenz um eine Aminosäuresequenz, die wenigstens den ersten 130 Aminosäuren des p23-Proteins des Epstein-Barr-Virus entspricht. Diese Aminosäuresequenz umfaßt den N-Terminus des p23-Proteins.

Ganz besonders bevorzugt sind solche autologen Fusionsproteine, bei denen die erste Aminosäuresequenz der Aminosäuresequenz des p23-Proteins des Epstein-Barr-Virus entspricht.

Die weitere (zweite) Aminosäuresequenz des Fusionsproteins entspricht der Aminosäuresequenz des serologisch (IF) als "Viruskapsid Antigen" des Epstein-Barr-Virus definierten späten Antigens p18 oder einem Teil dieses Viruskapsid Antigen-Proteins.

Eine weitere Aminosäuresequenz der erfindungsgemäßen autologen Fusionsproteine kann auch aus mehreren Teilen verschiedener Viruskapsid Antigenproteine bestehen, wobei die Viruskapsid-Antigene ausgewählt sind aus den Epstein-Barr-Virusproteiner mit den Bezeichnungen p150, p143, gp125, gp350, gp85, gp55, gp42, gp25, p40 und p18.

In einer besonders bevorzugten Ausführungsform handelt es sich bei der weiteren Aminosäuresequenz um eine Aminosäuresequenz, die dem bis zu 100 Aminosäuren langen C-Terminus des Proteins p18 des Epstein-Barr-Virus entspricht.

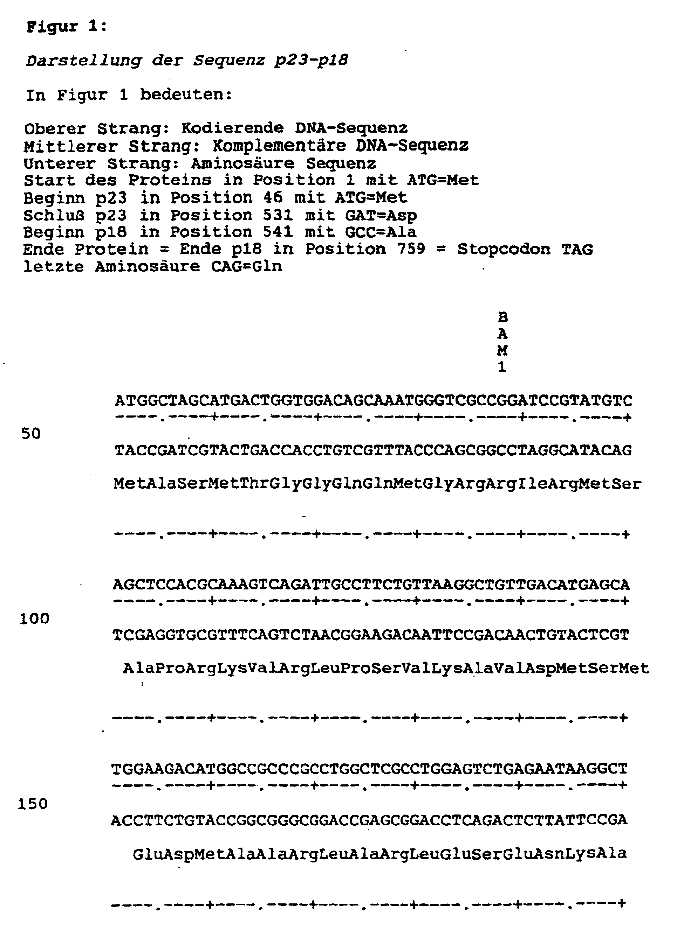

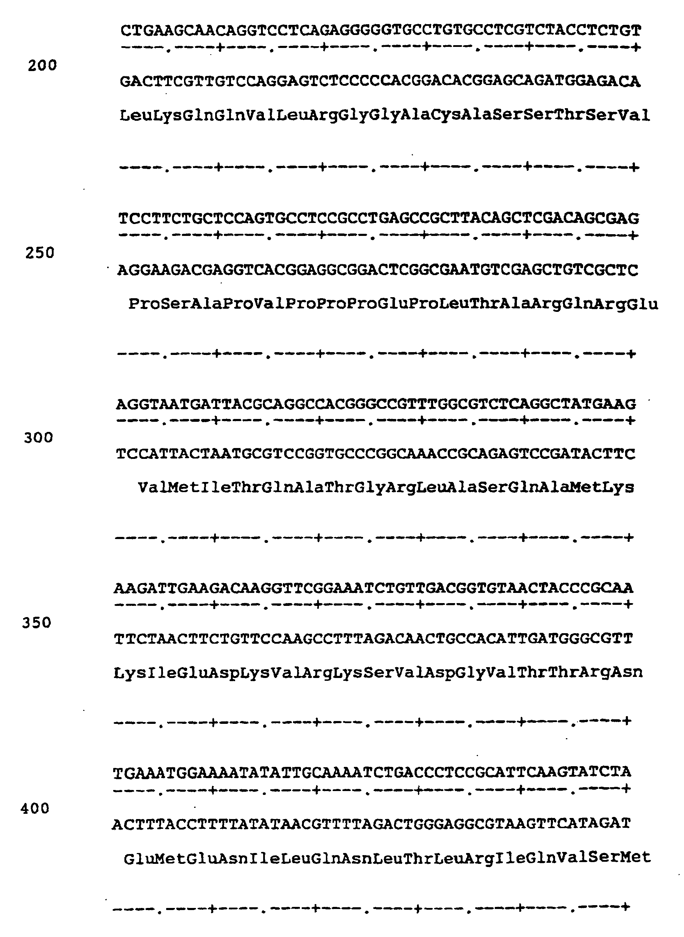

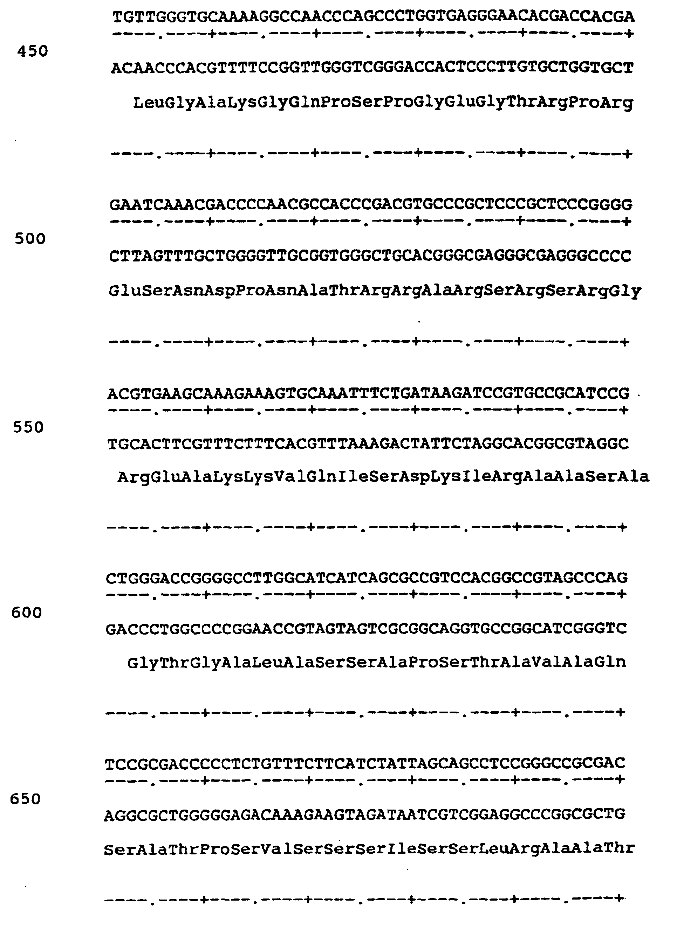

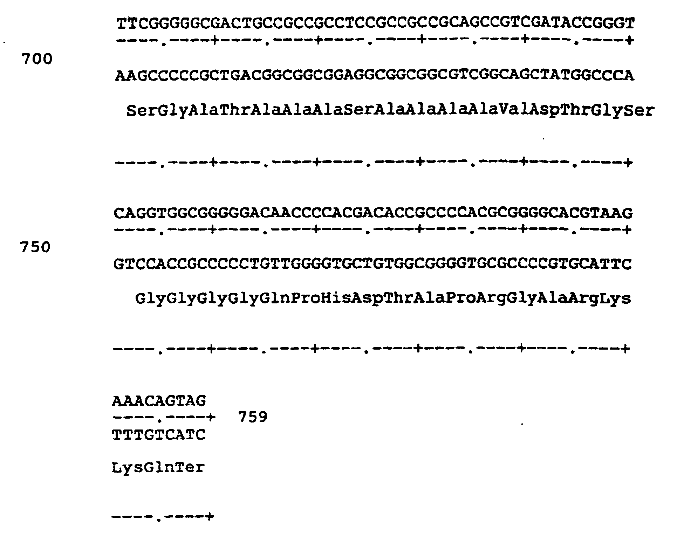

Ein im Rahmen der vorliegenden Erfindung besonders bevorzugtes autologes Fusionsprotein weist die Aminosäuresequenz gemäß Figur 1 auf.

Gegenstand der vorliegenden Erfindung sind auch Testkits zum Nachweis von Antikörpern, insbesondere von IgM- und/oder IgGoder auch IgA-Antikörpern gegen das Epstein-Barr-Virus, die wenigstens ein erfindungsgemäß autologes Fusionsprotein umfassen. Bevorzugt handelt es sich hierbei um Testkits zur Durchführung eines ELISA-Tests.

Die vorliegende Erfindung betrifft auch Verfahren zum Nachweis von Antikörpern, insbesondere von IgM- und/oder IgGsowie IgA-Antikörpern gegen das Epstein-Barr-Virus, bei denen man eine zu untersuchende Probe mit wenigstens einem erfindungsgemäßen Fusionsprotein zusammenbringt und den Antikörper-Fusionsprotein-Komplex mit einer Nachweiskomponente detektiert. In einer bevorzugten Ausführungsform ist die Nachweiskomponente ein Antihuman-Antikörper, der gekoppelt ist mit einem Indikatormolekül, wobei es sich bei dem Indikatormolekül um Meerrettich-Peroxidase handeln kann.

Die erfindungsgemäß offenbarten autologen Fusionsproteine weisen also wenigstens zwei Bestandteile auf. Der erste Bestandteil entspricht hinsichtlich der Aminosäure zumindest den 100 N-terminalen Aminosäuren des Proteins p23 des Epstein-Barr-Virus. Da dieser erste Teil mit einem weiteren Bestandteil fusioniert ist, der ebenfalls von dem Epstein-Barr-Virus herstammt, handelt es sich um ein autologes Fusionsprotein im Gegensatz zu einem heterologen Fusionsprotein, das einen Bestandteil aufweisen würde, der von einer anderen Quelle stammt. Die erfindungsgemäßen autologen Fusionsproteine können darüber hinaus noch einen kurzen Teil aufweisen, der bedingt ist durch die gentechnologische Herstellung des Fusionsproteins. Bei der Konstruktion des Vektors wird am N-Terminus eine Linkersequenz eingebaut, die für eine zufällige Aminosäuresequenz kodiert. Diese zufällige Aminosäuresequenz ist aber in immunologischer Hinsicht nicht relevant, da sie nicht einer natürlich vorkommenden Aminosäuresequenz entspricht. Zwischen den Bestandteilen des autologen Fusionsproteins können auch aufgrund der Aneinanderfügung der beiden Bestandteile Aminosäuren vorliegen, die aufgrund der Verbindung der beiden Bestandteile des Fusionsproteins entstanden sind. Auch dieser Bestandteil ist immunologisch nicht relevant.

Die erfindungsgemäßen autologen Fusionsproteine weisen am C-Terminus einen Bereich auf, der der Aminosäuresequenz eines anderen Proteins von Epstein-Barr-Virus oder einer Teilsequenz davon entspricht. Bei dieser weiteren Aminosäuresequenz handelt es sich um eine Aminosäuresequenz, die dem späten EBV Antigen p18 oder einer Teilsequenz davon entspricht. Im Rahmen der vorliegenden Erfindung sind auch die in Tabelle 1 näher beschriebenen späten EBV-Antigene einsetzbar:

**Tabelle 1**

| | | | |
|---|---|---|---|
| gibt die im Rahmen der vorliegenden Erfindung weiterhin verwendbaren späten EBV-Antigene an, die zu dem VCA-Komplex gehören. | | | |

| Späte EBV Antigene | | | |
|---|---|---|---|
| Protein | Lokalisation | Leserahmen | Serologie |
| p150 | Kapsid | BcLF1 | VCA-IgM? IgG schwach? |
| p143 | Tegument | BNRF1 | VCA? |
| p40 | Kapsid | BdRF1 | VCA-IgM/IgG? |
| p18 | Kapsid | BFRF3 | VCA-IgM, IgG spät |
| p23 | Kapsid? | BLRF2 | VCA-IgM, IgG spät |
| gp350 | Envelope | BLLF1 | MA, IgG spät |
| gp125 | ER? | BALF4 | VCA-IgM, IgG früh |
| gp85 | Envelope | BXLF2 | MA, VCA? |
| gp25 | Envelope | BKRF2 | MA, VCA? |
| gp42 | Envelope | BZLF2 | nicht bekannt |
| gp55 | Envelope? | BDLF3 | nicht bekannt |

Generell sind die EBV-Proteine nicht über das Molekulargewicht definiert. Da es keine einheitliche Nomenklatur gibt, haben sich die reading frame-Angaben nach Baer et al. durchgesetzt. Diesbezüglich wird auf die Veröffentlichung von Baer et al. verwiesen (Nature, Vol. 310, Juli 1984, 207), die eine genaue Charakterisierung der EBV-Proteine ermöglicht.

Das gp125 gilt als das Hauptimmunogen des serologisch definierten VCA-Komplexes, ist aber kein echtes Kapsid Antigen. Die Lokalisation ist unklar. Der Hauptnachteil von gp125 kann darin gesehen werden, daß eine potentielle Kreuzreaktivität zu anderen Herpesviren aufgrund der großen Homologien zu befürchten ist. Verwendbar sind daher nur solche Bereiche aus gp125, die keine Kreuzreaktivität aufzeigen, aber dennoch eine ausreichende Reaktivität besitzen. Ein weiterer Nachteil ist die schwere Herstellbarkeit dieses Proteins. Das Hauptkapsid Antigen p150 ist für sich allein genommen nicht reaktiv genug. Es hat nur eine begrenzte IgM-Reaktivität. Dies gilt gleichermaßen für das Hauptmembran Antigen gp350.

Die erfindungsgemäßen auto logen Fusionsproteine entsprechen hinsichtlich ihrer Aminosäuresequenz wenigstens zwei Bestandteilen, die unterschiedlichen Proteinen des Epstein-Barr-Virus entsprechen. Die erfindungsgemäßen autologen Fusionsproteine schließen auch Abwandlungen von Fusionsproteinen ein, bei denen hinsichtlich der Sequenz Deletionen, Substitutionen, Insertionen, Inversionen oder Additionen von Aminosäuren in der Sequenz vorliegen. Bei derartigen Veränderungen handelt es sich jedoch lediglich um solche, die die immunologischen Eigenschaften des Fusionsproteins nicht verändern. Es ist durchaus möglich, daß einzelne Aminosäuren durch in immunologischer Hinsicht gleichwirkende Aminosäuren ausgetauscht werden. Derartige Austausche sind insbesondere dann möglich, wenn Epitope nicht betroffen sind.

Die vorliegende Erfindung umfaßt auch Testkits zum Nachweis von Antikörpern, insbesondere IgG- und/oder IgM-Antikörpern gegen das Epstein-Barr-Virus. In der Diagnostik sind mehrere verschiedene Testmethoden wie Western Blot, Radioimmunoassay und andere Verfahren bekannt. Im Rahmen der vorliegenden Erfindung sind besonders solche Testkits bevorzugt, die sich zur Durchführung des ELISA-Tests (enzyme-linked immuno sorbent assay) eignen. Bei derartigen Tests findet eine Koppelung an eine feste Phase statt. Häufig wird dabei das Antigen, im vorliegenden Fall also das autologe Fusionsprotein, an eine feste Phase, insbesondere an die Mikrotiterplatten, gebunden und die freien Bindungsstellen werden abgesättigt. In diese Mikrotiterplatten wird dann die zu untersuchende Probe, meist Patientenserum, eingebracht, inkubiert und gewaschen. Die an die feste Phase gebundenen Antikörper sind dann gegen das nachzuweisende Antigen gerichtet. Diese Antikörper werden in der Regel mit Anti-Human-Immunglobulin-Antikörper nachgewiesen, an die eine Anzeigekomponente gebunden ist. Als besonders geeignet hat sich hierbei die Meerrettich-Peroxidase erwiesen, die eine Farbreaktion katalysiert, anhand der das Testergebnis abgelesen werden kann.

Ein alternatives Verfahren zum Nachweis spezifischer IgM-Antikörper stellt das µ-capture ELISA dar. Dabei wird die feste Phase eines Trägers, z.B. einer ELISA-Platte, mit gegen die µ-Kette des humanen IgM-Moleküls gerichteten polyklonalen bzw. monoklonalen Antikörpern beschichtet. Bei der nachfolgenden Inkubation mit humanem Serum binden IgM-Antikörper selektiv an die feste Phase, während Antikörper aller anderen Antikörperklassen durch Waschen entfernt werden. Die Bestimmung der EBV-spezifischen IgM-Antikörper aus der Vielzahl der gebundenen IgM-Moleküle erfolgt mittels eines enzymmarkierten EBV-Antigens, das durch die gegen dieses Antigen gerichteten IgM-Moleküle gebunden wird. Der gebildete Antikörper-Antigen-Komplex kann durch eine enzymvermittelte Farbreaktion nachgewiesen werden.

Anstelle einer Direktmarkierung des Antigens kann alternativ der Nachweis von EBV-spezifischen Antikörpern in der Untersuchungsflüssigkeit auch dadurch erfolgen, daß ein weiterer, für das Antigen spezifischer und markierter Antikörper, der bevorzugt ein mAK ist, zugegeben wird.

Die erfindungsgemäßen autologen Fusionsproteine weisen den Vorteil auf, daß sie in verhältnismäßig einfach durchzuführenden Testsystemen eine sehr hohe Spezifität und eine hohe Reaktivität aufweisen. Es ist möglich, bei den Tests zwischen IgM-, IgA- und IgG-Antikörpern zu unterscheiden, wobei lediglich der Anti-Human-Antikörper einmal gegen IgG- bzw. IgA-Antikörper und einmal gegen IgM-Antikörper gerichtet sein muß.

Ein überraschender Vorteil der erfindungsgemäßen autologen Fusionsproteine ist, daß diese sehr gut exprimiert werden können. Häufig tritt bei der Expression von Fremdproteinen in Wirtszellen die Schwierigkeit auf, daß diese nicht oder nur in sehr geringen Mengen exprimiert werden. Um diese Schwierigkeit zu umgehen, wurden verschiedene Vektoren konstruiert, die die Expression von heterologen Fusionsproteinen ermöglichen. Grundsätzlich weisen die exprimierten heterologen Fusionsproteine einen Anteil auf, der von dem Vektor herstammt, und einen anderen Anteil, der das gewünschte Peptid bzw. Protein darstellt. Häufig verwendet werden hierbei im E.coli-System Vektoren, die die Glutathion-S-Transferase oder die Beta-Galaktosidase oder Teile dieser Proteine exprimieren. Nachteilig bei diesen heterologen Fusionsproteinen ist, daß es in den Seren Antikörper geben kann, die gegen den vom Vektor herstammenden Teil gerichtet sind, also gegen die Glutathion-S-Transferase (GST) oder die Beta-Galaktosidase. Wenn derartige Antikörper in den Seren vorliegen, wird ein falsch positives Resultat erhalten.

Cartwright et al. [J. Immunol. Meth. 179 (1995) 31-35] beschreibt einige Probleme, die bei der Verwendung von GST-Fusionsproteinen bei immunologischen Tests, insbesondere ELISA-Tests, auftreten.

Andererseits ist es in der Regel sehr schwierig, heterologe, insbesondere virale Proteine in E.coli in ausreichender Ausbeute zu erhalten, wenn die Expression nicht als heterologes Fusionsprotein erfolgt. Die vorliegende Erfindung ermöglicht aber überraschenderweise eine sehr gute Expression der autologen Fusionsproteine auch ohne heterologen Fusionsanteil.

In bevorzugter Ausführungsform wurde hierbei das "pET-Vektorsystem" verwendet, das von Studier et al. in Methods Enzymol. 185, (1990), 60-89 beschrieben wurde.

Ein weiterer unerwarteter Vorteil der erfindungsgemäßen autologen Fusionsproteine ist die leichte Reinigung, die aufgrund von Eigenschaften der autologen Fusionsproteine ermöglicht wird. Nach der Expression der autologen Fusionsproteine in den Wirtszellen liegen die autologen Fusionsproteine in unlöslicher Form bei einem pH-Wert von etwa 7,5 vor. Aufgrund der Unlöslichkeit können die hergestellten autologen Fusionsproteine sehr gut von den anderen aus dem Wirtsorganismus stammenden Proteinen abgetrennt werden. Durch eine Veränderung der Salzkonzentration und Erhöhung des pH-Wertes auf etwa pH 9 können die exprimierten autologen Fusionsproteine jedoch wieder in Lösung gebracht werden. Diese überaschende Eigenschaft ist wahrscheinlich auf die besondere Struktur des Proteins p23 zurückzuführen.

Es ist aber auch möglich, als autologe Fusionsproteine gemäß der vorliegenden Erfindung solche Proteine herzustellen, die auf andere Art und Weise praktisch nicht gentechnologisch darstellbar sind. Hierbei ist insbesondere ein sehr schwer zu exprimierender Bereich aus dem p54-Protein (BMRF1) zu erwähnen.

Die vorliegende Erfindung wird durch die nachfolgenden Beispiele näher erläutert.

### Beispiel 1

### Klonierung des autologen Fusionsproteins 23/1 18/1

Es handelt sich hier um ein autologes Fusionsprotein, das den kompletten Bereich von p23 und die 72 C-terminalen Aminosäuren von p18 kombiniert.

### Vorgehensweise:

1. Ausgehend von einem Cosmid-Klon CM 302-23, der einen Teilbereich des EBV-Genoms enthält, erfolgte eine PCR-Amplifikation des DNA-Fragmentes PCE23/1, mit Hilfe geeigneter Primer. Beide Primer besitzen zusätzlich zum p23 komplementären Bereich Überhänge, die bestimmte singuläre Restriktionsschittstellen enthalten, d.h PCE232.seq: EcoRI, PCE231.seq: BamHI. Diese Schnittstellen ermöglichen die gerichtete Klonierung des Amplifikats. Die PCR-Amplifikation erfolgte in einem Gesamtvolumen von 100 µl bestehend aus 10µl Reaktionspuffer (Perkin-Elmer Cetus), 200µM der 4 Desoxynucleotide, 0,5µM beider PCR-Primer, 50-100ng der Ausgangs-DNA sowie 2,5 Units der AmpliTaq DNA-Polymerase (Perkin-Elmer Cetus). Die Amplifikation wurde im Perkin-Elmer Cetus DNA Thermal Cycler in 25 Zyklen bei folgenden Bedingungen durchgeführt: 1 min - 55°C, 1 min - 72°C, 1 min - 94°C. Der PCR-Reaktionsansatz wurde in einer Submarine-Agarose Gelkammer in einem 1,2 %igem Agarosegel (Ultra Pure Agarose, BRL), das 0,5 µg/ml Ethidiumbromid enthält unter Verwendung eines TBE- Laufpuffers (0,089M Tris/Borat, 0,002M EDTA) elektrophoretisch aufgetrennt. Danach wurde das amplifizierte DNA-Fragment mit einer UV-Leuchte bei 364 nm sichtbar gemacht und der Gelbereich, der die entsprechende Bande enthielt, mit einem Skalpell herausgeschnitten. Die Elution der DNA aus dem Gelfragment erfolgte mittels einer Biotrap Kammer (Schleicher & Schüll) entsprechend der Vorschrift des Herstellers. Daran schloß sich eine zusätzliche Aufreinigung der DNA mit einer Elutip D-Säule (Schleicher & Schüll) entsprechend der Gebrauchsanweisung des Herstellers an. Das so gereinigte und getrocknete DNA-Fragment wurde in 80 µl aqua dest gelöst. Nach Zugabe von 10 µl NEBuffer 4 (New England Biolabs) erfolgte ein BamHI/EcoRI Verdau durch Zugabe von jeweils 100 U beider Enzyme. Nach 2 Stunden folgte eine Phenol-Extraktion mit anschließender Ethanolprezipitation. 100ng des so vorbereiteten DNA-Fragments wurden mit 200 ng BamHI/EcoRIbehandeltem DNA des Standardvektors puc8 für 16h bei 4°C ligiert und anschließend damit E.coli JM109 transformiert. Die Ausplattierung des Transformationsansatzes erfolgte auf mit Ampicillin (50µg/ml) und X-Gal(30µg/ml) versetzten Agarplatten. Weiße Kolonien wurden in 3 ml LB-Medium mit Ampicillin überimpft und für 12-16h bei 37°C unter Schütteln inkubiert. Nach Isolierung der Plasmid-DNA erfolgte ein EcoRI/BamHI Restriktionsverdau und eine Elektrophorese im Agarosegel. Ein Klon, bei dem sich ein zusätzliches DNA-Fragment der erwarteten Größe zeigte, erhielt den Namen puc8/PCE23/1 und wurde zur weiteren Klonierung verwendet. DNA des Klons puc8/PCE23/1 wurde mit den Restriktionsenzymen AvaI und EcoRI behandelt. Der so geöffnete Vektor wurde mit je 100ng der beiden Oligonukleotide PCE23F1.seq und PCE23F2.seq ligiert und anschließend in JM 109 wie oben beschrieben transformiert. Nach Isolierung der Plasmid-DNA erfolgte ein Bgl II-Verdau. Ein Klon, bei dem eine Liniarisierung des Vektors festzustellen war, erhielt den Namen puc8/PCE23F/1.
2. Ausgehend vom Cosmid-Klon CMB-14 erfolgte PCR-Amplifikation mit Hilfe der Primer PCC181.seq und PCE182.seq und die spätere Klonierung des amplifizierten Fragments wie oben beschrieben. Der entsprechende Klon wurde als puc8/PCE18/1 bezeichnet.
3. Mit DNA des Klons puc8/PCE18/1 erfolgte ein Restriktionsverdau mit BamHI und EcoRI. Das dabei freigesetzte Fragment von ca. 230 bp wurde mit Hilfe einer Agarose-Elektrophorese isoliert, und wie oben beschrieben aus dem herausgeschnittenen Gelstück eluiert. Ca. 50ng dieses DNA-Fragmentes wurden dann mit 200ng des mit BglII und EcoRI geöffneten und ebenfalls mittels Elektrophorese gereinigten Vektors puc8/PCE23F1 ligiert. Daran schloß sich eine Transformation mit E.coli JM109 and eine Ausplattierung auf Ampicillin enthaltenden Agarplatten an. Isolierte Kolonien wurden in 3ml LB-Medium mit Ampicillin überimpft und 12-16h unter Schütteln bei 37°C inkubiert. Nach Isolierung der Plasmid-DNA erfolgte ein BamHI/EcoRI-Restriktionsverdau mit anschließender Elektrophorese im Agarosegel. Ein Klon mit einem Insert der erwarteten Größe wurde als puc8/ 23/1 18/1 bezeichnet und für die weiteren Experimente verwendet. Bei dieser Vorgehensweise wird ausgenutzt, daß die Erkennungssequenzen von BamHI und BglII die gleichen Überhänge besitzen, wodurch eine Ligation ermöglicht wird. Beide Schnittstellen gehen jedoch nach erfolgter Ligation verloren. Der Übergang von 23/1 zu 18/1 ist so gewählt, daß eine Translation im gleichen Leserahmen möglich ist. Figur 1 zeigt die gesamte DNA-Sequenz des oben beschriebenen Konstrukts und die entsprechende AS-Sequenz des resultierenden autologen Fusionsproteins.
4. Um die Expression des autologen Fusionsproteins zu gewährleisten, erfolgte die Umklonierung des entsprechenden DNA-Fragmentes in den Vektor pET5c, der den starken T7-Promotor des Gens 10 des Bakteriophagen T7 besitzt. Dahinter besitzt dieser Vektor eine Ribosomenbindungstelle und ein Startcodon in entsprechendem Abstand. Hinter dem Startcodon liegt ein Leserahmen von 11 Aminosäuren des Gens 10 des Bakteriophagen T7 und eine BamHI und eine EcoRI-Schnittstelle. Das Leseraster der BamHI-Schnittstelle stimmt mit der des oben beschriebenen autologen Fusionsproteins überein. 100ng des mittels EcoRI- und BamHI-Restriktionsverdau aus puc8/23/1 18/1 freigesetzte DNA-Fragments wurden mit 200ng des mit den gleichen Restriktionsenzymen geöffneten Vektor für 16h bei 4°C ligiert. Danach folgte eine Transformation mit E.coli JM109 und eine Ausplattierung auf Ampicillin-haltigen Platten. Isolierte Kolonien wurden in 3ml LB-Medium mit Ampicillin überimpft und 16h unter Schütteln bei 37°C inkubiert. Nach Isolierung der Plasmid-DNA erfolgte ein BamHI/EcoRI-Restriktionsverdau. Ein Klon mit einem Insert der zu erwartenden Größe wurde als pET5c/ 23/1 18/1 bezeichnet. Um die Expression des rekombinanten Proteins zu gewährleisten, wurde DNA des Expressionsvektors in den chloramphenicolresistenten Expressionsstamm BL21(DE3)pLysS transformiert. Einer der resultierenden Klone wurde für die weiteren Arbeiten verwendet.

### Beispiel 2

### Anzucht, Expression und Reinigung des autologen Fusionsproteins p23 - p18

Ausgehend von einer Plattenkolonie des Klons pET5c/p23/1 p18/1/BL21(DE3)pLysS wurde eine 15 ml Kultur bis zu einer optischen Dichte bei 600 nm von etwa 2,0 angezogen. Die DNA-Sequenz des autologen Fusionsproteins und die davon abgeleitete Aminosäuresequenz ist in Figur 1 dargestellt. Die Kultur wurde mit Glycerin (87 %) bis zu einer Endkonzentration von 15 % (v/v) vermischt und dann in 0,1 ml-Portionen eingefroren und bei -60 bis -80°C tiefgefroren.

Ein gefrorenes Aliquot der Vorkultur wurde in 150 ml LB/CA, AMP-Medium angezogen und dann in einen größeren Ansatz überführt. Bei einer A (600 nm) von 0,6 wurde durch Zugabe von IPTG (Endkonzentration 1 mM) induziert.

Drei Stunden nach Induktion wurde durch Zentrifugation geerntet, die abzentrifugierten Bakterienpellets wurden mit PBS-Puffer gewaschen und nach abermaliger Zentrifugation eingefroren und bei -20 bis -30°C gelagert.

Die eingefrorenen Bakterienpellets wurden aufgetaut und in 160 ml Tris-HCl/20 mM, pH 7,5 resuspendiert und anschließend homogenisiert. Unter Rühren bei Raumtemperatur wurden folgende Additive zugegeben: NP-40 (0,05 %), PMSF (0,2 mM), Pefabloc (0,2 mM), EDTA (50 mM) und Lysozym (50 mg). Das Gesamtvolumen betrug 200 ml. Der Ansatz wurde nach Lysozymzugabe sofort auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 280 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 10 Stöße). Das solubilisierte Material wurde durch Zentrifugation entfernt; die Überstände wurden verworfen.

Die Pellets wurden gut abgetropft und sofort resuspendiert in 100 ml Basis-Puffer mit zusätzlich 500 mM NaCl, 0,5 % NP-40 und 14 mM Mercaptoethanol und anschließend mit einem Teflon/Glas-Potter-Homogenisator (1000 UpM, 3 Stöße) homogenisiert. Der Ansatz wurde anschließend sonifiziert (20 kHz, pulsierend, 5 min, Titanrüssel 3/4"). Solubilisiertes Material wurrde durch Zentrifugation entfernt und die Überstände wurden verworfen.

Die Pellets wurden nun gut abgetropft und in 200 ml Tris-HCl/20 mM/pH9 mit 14 mM Mercaptoethanol und mit einem Teflon/Glas-Potter-Homogenisator (1000 UpM, 3 Stöße) homogenisiert. Der Ansatz wurde dann noch sonifiziert (20 kHz, pulsierend, 5 min, Titanrüssel 3/4"). Nichtsolubilisiertes Material wurde durch Zentrifugation entfernt. Die Sedimente wurden verworfen und die Überstände vereinigt und eingefroren und über Nacht gelagert bei -20° bis -30°C.

Die solubilisierte Fraktion wurde aufgetaut und es wurde unter Rühren im Eisbad festes, fein gemörsertes Ammoniumsulfat langsam zugegeben (innerhalb 15 min) bis zu einer Konzentration von 25 % Sättigung. Es wurde noch 15 min weitergerührt. Anschließend wurde zentrifugiert (2 x 250 ml-Becher, 27000 g, 30 min, 0-4°C). Die Pellets wurden verworfen. Der Überstand wurde erneut innerhalb 15 min mit Ammoniumsulfat versetzt, bis zu einer Konzentration von 40 % Sättigung, nochmal für 15 min gerührt und wie vorher zentrifugiert. Der Überstand wurde verworfen. Die Pellets wurden resuspendiert in 25 ml Tris-HCl/20 mM/pH9, mit zusätzlich 2-Mercaptoethanol (14 mM), Pefabloc (0,1 mM) und Glycerin (10 %), eingefroren und über Nacht gelagert bei -2° bis -30°C.

Die mit Ammoniumsulfat (25-40 %) fraktionierte Protein-Lösung wurde aufgetaut, und präzipitiertes Protein wurde entfernt durch Zentrifugation (50 ml-Becher, 40000 g, 30 min, 0-4°C). Der Überstand wurde mit Milli-Q-Wasser auf das 3-fache Volumen verdünnt und über SP-Sepharose (Fast Flow) chromatographiert. Dabei wurde eine Säule der Dimension 2,6 x 12 cm (60 ml) verwendet. A (280 nm) und die Leitfähigkeit wurden kontinuierlich registriert. Der Fluß betrug 5 ml/min. Säulenpuffer war Tris-HCl/20 mM/pH9,5 mit 2-Mercaptoethanol (1,4 mM). Nach Probenauftrag, gefolgt von 100 ml Säulenpuffer, wurde zunächst ein flacher linearer NaCl-Gradient (dC/dV = 3 mM/ml, bis 0,9 M) gefolgt von einem steileren NaCl-Gradient (dC/dV = 6 mM/ml) in Säulenpuffer angelegt. Das Eluat wurde in 10 ml-Fraktionen zerlegt und eingefroren. Die Antigen-enthaltenden Fraktionen fanden sich im Bereich zwischen 400-700 mM NaCl. Der Nachweis erfolgte durch SDS-PA-Disc-Elektrophorese mit anschließender Coomassie-Färbung.

Die Fraktionen, die reines p23-p18-Antigen enthielten (Coomassie-Gel), wurden aufgetaut, vereint, mit Glycerin versetzt bis zu einer Endkonzentration von 20 % und mittels Ultrafiltration (Rührzelle, Eisbad, Membran = Omega 30) konzentriert bis zu einer Proteinkonzentration von mindestens 5 mg/ml. Das Retentat wurde der Kammer entnommen und aliquotiert eingefroren und gelagert bei -60 bis -80°C.

### Beispiel 3 (Vergleichsbeispiel)

### Anzucht, Expression und Reinigung des Proteins p23

Anzucht, Expression, Lyse und die weitere Reinigung erfolgte wie in Beispiel 1 beschrieben. Ausgangsklon war hierbei das Konstrukt pET5c/p23/1/BL21(DE3)pLysS.

### Beispiel 4 (Vergleichsbeispiel)

### Anzucht, Expression und Reinigung des heterologen Fusionsproteins GST-p18

Mit einer isolierten Kolonie auf einer Agarplatte des Klons pGEX-3/p18/1/JM109 (LB/AMP Medium) wurden 150 ml LB/AMP-Medium angeimpft. Die Kultivierung erfolgte in einem 11 EMK, bei 37°, im Rundschüttler bei 160 Upm für 16 h.

Die Anzucht der 3 l Kultur erfolgte bei 37°C und 160 Upm im Rundschüttler. Das Wachstum wurde kontinuierlich verfolgt durch Messung der Absorption A bei 600 nm. Bei einer A (600 nm) von 0,7 wurde induziert durch Zugabe von IPTG (Endkonz. 1 mM).

Die Ernte erfolgte 4 h nach Induktion durch Zentrifugation. Die gut abgetropften Bakterienpellets wurden in 200 ml eiskaltem PBS resuspendiert und erneut zentrifugiert und dann eingefroren und gelagert bei -20 bis -30°C.

Nach Auftauen der Bakterienpellets wurden diese in 80 ml Basis-Puffer (Tris-HCl/20mM/pH7,5) resuspendiert und anschließend homogenisiert (Teflon/Glas-Potter-Homogenisator). Unter Rühren bei Raumtemperatur wurden folgende Additive zugegeben: NP-40 (0,1 %), PMSF (0,1 mM), Pefabloc (0,1 mM), EDTA (50 mM) und Lysozym (100 mg). Das Gesamtvolumen betrug 100 ml. Der Ansatz wurde bei Raumtemperatur stark gerührt und nach 60 min auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 140 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 10 Stöße). Nichtsolubilisiertes Material wurde durch Zentrifugation abgetrennt und verworfen.

Das lösliche Protein des Überstands wurde über GSH-Sepharose-4B (Pharmacia) chromatographiert. Dabei wurde eine Säule der Dimension 2,6 x 10 cm (30 ml) verwendet. A (280 nm) wurde kontinuierlich registriert. Die Flußrate betrug 2 ml/min. Der Säulenpuffer war Tris-HCl/20 mM/pH7,5 mit 1,4 mM Mercaptoethanol. Nach erfolgtem Probenauftrag wurde solange mit Säulenpuffer nachgespült, bis der Schreiber die Basislinie wieder erreicht hatte. Anschließend wurden 50 ml Waschpuffer, Tris-HCl/100 mM/pH9 mit 10 % Glycerin, 0,5 % NP-40 und 1,4 mM Mercaptoethanol über die Säule gegeben gefolgt von Säulenpuffer. Nachdem der Schreiber wieder die Basislinie erreicht hatte, wurde das gebundene GST-Protein mit 5 mM Glutathion (GSHred.) in Säulenpuffer eluiert. Das GSH wurde unmittelbar vor der Verwendung gelöst. Das GSH-Eluat (vom Schreiber registrierter Absorptionspeak) wurde gesammelt und aliquotiert eingefroren und gelagert bei -60 bis -80°C.

### Beispiel 5

### Reakti vität der gereinigten rekombinanten Antigene im ELISA

### 1. Testdurchführung

Je 100ng des gereinigten rekombinanten Antigens gelöst in 100µl 0,01 M Carbonatpuffer pH 9,5 wurden in die Vertiefungen von Mikrotiterplatten (Nunc) gegeben und 16h in einer geerdeten feuchten Kammer inkubiert. Nach Zugabe von 100µl einer Kalbserum enthaltenden Nachbeschichtungslösung wurde die Inkubation für 2 weitere Stunden fortgesetzt. Danach wurden die Platten entleert und sorgfältig ausgeschlagen. Anschließend wurden die Platten für die eigentliche Testdurchführung verwendet oder nach Trocknung im Vakuumschrank und anschließendem Einschweißen in Folienschlauch bis zum späteren Gebrauch bei -20°C gelagert. Zur eigentlichen Testdurchführung wurden die Testnäpfchen der ELISA-Platten mit 100µl 1:21 verdünnten Serum gefüllt, mit einer Plastikfolie abgeklebt und 1h bei 40°C schwimmend im Wasserbad inkubiert. Nach dreimaligem Waschen im Biotest ELISA Washer II erfolgte die Inkubation mit 100µl von gegen humanes IgG bzw. IgM gerichteten, Peroxidase-markierten, monoklonalen Antikörpern aus der Maus für 30min (IgG) bzw. 60min (IgM) bei 40°C. Nach abermaligen Waschen wurden die gebundenen Antikörper durch eine Farbreaktion mit 1,2-Phenyldiamin als Chromogen sichtbar gemacht. Die optische Dichte der einzelnen Probe wurde bei 495nm (Referenz: 620nm) am Anthos HTII ELISA-Reader ermittelt. Alle OD-Werte größer als 0,3 wurden als positives Ergebnis gewertet.

### Beispiel 6

### Kupplung von gereinigtem p23/18-Antigen mit meerrettich-Peroxidase (POD) und Nachweis von virusspezifischen Antikörpern mit p23/18-POD als löslichem Liganden im ELISA

Zur POD-Markierung wurde das gereinigte p23/18-Antigen in Carbonatpuffer (100mM, NaCO3, PH 9,5, 20% Glycerin) aufgenommen (Lösung A). Die Meerrettich-Peroxidase wurde mit Natriumperiodat nach der Methode von Nakane und Kawaoi (J. Histochem. & Cytochem., Vol. 22, No. 12, pp. 1084-1091) aktiviert. Die Abtrennung von freiem NaIO₄ erfolgte durch Chromatographie über Sephadex G25 coarse (Pharmacia). Die Säule (XK26, Pharmacia) umfaßte ein Volumen von 150 ml und wurde mit einer Flußrate von 2 ml/min beladen. Nach dem Säulenlauf lag die aktivierte POD im Elutionspuffer (10mM NaCO3, PH 9,5) vor (Lösung B). Zur Kupplung wurden 0,59 ml Lösung A (1 mg Antigen) und 6,9 ml Lösung B (6 mg POD) in einen Erlenmeyerkolben (EMK) pipettiert und bei Raumtemperatur (RT) für 2 h inkubiert. Der Reaktionsansatz wurde auf 4°C abgekühlt, mit 300 µl NaBH₄ (5 mg/ml Elutionspuffer) versetzt und für weitere 18 - 24 h bei 4°C aufbewahrt. Nach der Kühllagerung wurden dem Gemisch 30 µl Aceton zugegeben und 30 - 40 min bei RT inkubiert. Zur Stabilisierung des Reaktionsprodukts (p23/18-POD) wurden 2,1 ml einer Albumin-Lösung (14,3 mg/ml BSA, 0,85mM NH₄FeIISO₄, 0,01 M PBS, PH 7,2) hinzugefügt, der Reaktionsansatz aliquotiert und bei -70°C gelagert (Lösung C). Die Effizienz der Kupplung wurde durch SDS-Gelelektrophorese und durch den Nachweis der Peroxidaseaktivität des POD-markierten Antigens überprüft.

Die immunologische Aktivität des POD-markierten p23/18-Antigens konnte im enzyme immunoassay (ELISA) nachgewiesen werden. Das Testverfahren basierte auf den Prinzipien eines "capture-Tests". Dazu wurden Mikrotiterplatten mit einem monoklonalen Antikörper, der gegen die konstante Kette von human IgM gerichtet ist (MAKµ), beschichtet. Die Konzentration der Beschichtungslösung (100 µl/well) betrug 10 µg MAKµ/ml Carbonatpuffer (10mM NaCO₃, 0,1% NaN₃, PH 9,5). Die Seren (Serum 1 - 19) von gesunden Blutspendern und die Seren (Serum 20 - 30) von Patienten mit akuter Primär- oder reaktivierter EBV-Infektion wurden als Proben verwendet. Die Proben wurden 1:21 im Probenpuffer verdünnt. Jeweils 200 µl des Gemisches wurden nach dem Dekantieren der Beschichtungslösung pro Napf pipettiert und für 1 h bei 37°C inkubiert. Danach wurde die Mikrotiterplatte ausgesaugt und dreimal gewaschen. Der verwendete Waschpuffer wurde im Verhältnis 1:10 mit 0,9% NaCl-Lösung versetzt. Danach wurde das POD-markierte p23/18-Antigen (Lösung C) 1:1000 in Probenpuffer verdünnt und 100 µl pro Napf pipettiert. Die Reaktion wurde nach 1 h bei 37°C durch Waschen der Mikrotiterplatte beendet. Das gefundene p23/18-Konjugat wurde durch eine Farbreaktion mit 1,2-Phenylendiamin und H₂O₂ nachgewiesen. Die optische Dichte (OD) der einzelnen Proben wurde bei 495 nm (Referenz 620 nm) am Anthos HTII ELISA-Photometer ermittelt. Alle OD-Werte >0,3 wurden als positiv gewertet.

Die Tabelle 2 zeigt die OD-Werte, die für die verwendeten Serenproben ermittelt wurden. Als Referenz wurde ein ELISA nach dem "sandwich-Prinzip" durchgeführt. Das p23/18-Antigen stellte dabei den Festphasenrezeptor dar und anti-human IgM MAKµ-POD wurde als löslicher Ligand verwendet. Seren, die einen OD-Wert >0,150 - NK (cut off) aufweisen, sind als positiv, OD-Werte unterhalb des cut off's sind als negativ zu werten. Von den 19 Seren, die keine EBV-spezifischen IgM-Antikörper enthielten, wurden 19 in beiden Testverfahren als negativ erkannt. Alle Seren mit primären oder reaktivierten EBV-IgM Antikörpern wurden in beiden Tests als positiv detektiert. Zwischen den beiden Tests ergeben sich bei dem eingesetzten Probenumfang keine diskrepanten Ergebnisse. Die ermittelten OD-Werte zeigen, daß das p23/18-POD Konjugat als löslicher Ligand im "capture-Test" eingesetzt werden kann und seine immunologischen wie enzymatischen Aktivitäten beibehält.

Lagerungsversuche mit p23/18-POD als Gebrauchsverdünnung zeigten eine sehr gute Stabilität, des Antigen-Enzym Konjugats. Es ergaben sich keine wesentlichen Abnahmen der OD-Werte nach 3 Tagen Lagerung bei -20°C, 4°C, 37° und 50°C.

**Tabelle 2:**

| OD-Werte für die verwendeten Serenproben | | | | | |
|---|---|---|---|---|---|
| **Seren** **NK** **PK** | **p23/18** **sandwich** **Test** **0,020** **1,473** | **p23/18** **capture-Test** **0,048** **1,107** | **Seren** | **p23/18** **sandwich** **Test** **0,020** **1,473** | **p23/18** **capture-Test** **0,048** **1,107** |
| 01 | 0,082 | 0,053 | 16 | 0,022 | 0,040 |
| 02 | 0,089 | 0,042 | 17 | 0,011 | 0,047 |
| 03 | 0,025 | 0,048 | 18 | 0,091 | 0,058 |
| 04 | 0,034 | 0,049 | 19 | 0,027 | 0,040 |
| 05 | 0,028 | 0,051 | 20 | 1,327 | 2,166 |
| 06 | 0,032 | 0,049 | 21 | 0,980 | 1,224 |
| 07 | 0,014 | 0,060 | 22 | 0,930 | 1,030 |
| 08 | 0,035 | 0,059 | 23 | 1,456 | 0,490 |
| 09 | 0,027 | 0,080 | 24 | 1,723 | 1,708 |
| 10 | 0,023 | 0,093 | 25 | 2,476 | > 3 |
| 11 | 0,003 | 0,077 | 26 | 2,566 | 2,777 |
| 12 | 0,165 | 0,088 | 27 | 2,415 | 1,364 |
| 13 | 0,107 | 0,044 | 28 | 2,476 | > 3 |
| 14 | 0,003 | 0,035 | 29 | 1,327 | > 3 |
| 15 | 0,026 | 0,033 | 30 | 2,530 | > 3 |

### Beispiel 7

### Vergleich der serologischen Reaktivität

Die in Beispiel 5 näher erläuterte Testdurchführung wurde zur Überprüfung der serologischen Reaktivität der Proben GST-p18, p23 und p23-p18 verwendet. Es wurden einerseits 22 Patienten mit akuter EBV-Primärinfektion getestet und andererseits wurden 105 freiwillige Blutspender überprüft, wobei es sich hierbei um gesunde Stamm-Blutspender des DRK-Frankfurt handelt.

Die Ergebnisse des Tests sind in der nachfolgenden Tabelle 3 dargestellt.

**Tabelle 3**

| Anzahl und Rate (%) Positiver | Patienten (n = 22) | | Blutspender (n = 105) | |
|---|---|---|---|---|
| | IgM | IgG | IgM | IgG |
| GST-p18 | 22 (100.0) | 10 (45.5) | 2 (1.9) | 96 (91.4) |
| p23 | 4 (18.2) | 14 (63.6) | 0 (0.0) | 68 (64.8) |
| p23-p18 | 22 (100.0) | 15 (68.2) | 1 (1.0) | 99 (94.3) |

Die Tabelle 3 belegt folgende überraschende Vorteile des erfindungsgemäßen Fusionsproteins:
a) IgG-Serologie:
   Der mit dem Fusionsprotein p23-p18 durchgeführte IgG-ELISA war sensitiver als der mit p23 bzw. GST-p18 durchgeführte ELISA. Das Fusionsantigen erkannte gegenüber GST-p18 drei zusätzliche Blutspender und (bei den Patienten) fünf zusätzliche Primärinfektionen. Diese Seren waren offensichtlich aufgrund des p23-Anteils positiv.
   Gegenüber p23 (allein) erkannte das erfindungsgemäße Fusionsprotein 31 zusätzliche Blutspender und eine zusätzliche Primärinfektion bei den Patienten. Die überlegene Sensitivität bei abgelaufenen Infektionen wie auch bei Primärinfektionen des erfindungsgemäßen Infusionsproteins konnte nicht vorhergesehen werden.
b) IgM-Serologie:
   Gegenüber GST-p18 besitzt das erfindungsgemäße Fusionsantigen eine verbesserte Spezifität. Bei den Blutspendern war ein falsch positiver Fall auf IgM-Antikörper gegen den GST-Anteil zurückzuführen. Die autologe Fusion von p18 mit p23 verbesserte also wesentlich die Spezifität gegenüber der heterologen Fusion mit GST.

### SEQUENZPROTOKOLL

(1) ALLGEMEINE INFORMATION:
   ANMELDER:
      (A) NAME: Biotest AG
      (B) STRASSE: Landsteinerstr. 5
      (C) ORT: Dreieich
      (E) LAND: Germany
      (F) POSTLEITZAHL: 63303
   ANMELDETITEL:
      Rekombinante autologe Fusionsproteine des
      Epstein-Barr-Virus, diese enthaltende
      Testkits und Verfahren zum Nachweis...
   ANZAHL DER SEQUENZEN: 1
   COMPUTER-LESBARE FORM:
      (A) DATENTRÄGER: Floppy Disk
      (B) COMPUTER: IBM PC compatible
      (C) BETRIEBSSYSTEM: PC-DOS/MS-DOS
      (D) SOFTWARE: PADAT Sequenzmodul Version 1.0
(2) INFORMATION ZU SEQ ID NO: 1:
   (i) SEQUENZ CHARAKTERISTIKA:
      (A) LÄNGE: 759 Basenpaare
      (B) ART: Aminosäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 1:

ATG GCT AGC ATG ACT GGT GGA CAG CAA ATG GGT CGC CGG ATC CGT ATG 48
Met Ala Ser Met Thr Gly Gly Gln Gln Met Gly Arg Arg Ile Arg Met
1               5                   10                  15

TCA GCT CCA CGC AAA GTC AGA TTG CCT TCT GTT AAG GCT GTT GAC ATG 96
Ser Ala Pro Arg Lys Val Arg Leu Pro Ser Val Lys Ala Val Asp Met
            20                  25                  30

AGC ATG GAA GAC ATG GCC GCC CGC CTG GCT CGC CTG GAG TCT GAG AAT 144
Ser Met Glu Asp Met Ala Ala Arg Leu Ala Arg Leu Glu Ser Glu Asn
        35                  40                  45

AAG GCT CTG AAG CAA CAG GTC CTC AGA GGG GGT GCC TGT GCC TCG TCT 192
Lys Ala Leu Lys Gln Gln Val Leu Arg Gly Gly Ala Cys Ala Ser Ser
    50                  55                  60

ACC TCT GTT CCT TCT GCT CCA GTG CCT CCG CCT GAG CCG CTT ACA GCT 240
Thr Ser Val Pro Ser Ala Pro Val Pro Pro Pro Glu Pro Leu Thr Ala
65                  70                  75                  80

CGA CAG CGA GAG GTA ATG ATT ACG CAG GCC ACG GGC CGT TTG GCG TCT 288
Arg Gln Arg Glu Val Met Ile Thr Gln Ala Thr Gly Arg Leu Ala Ser
                85                  90                  95

CAG GCT ATG AAG AAG ATT GAA GAC AAG GTT CGG AAA TCT GTT GAC GGT 336
Gln Ala Met Lys Lys Ile Glu Asp Lys Val Arg Lys Ser Val Asp Gly
            100                 105                 110

GTA ACT ACC CGC AAT GAA ATG GAA AAT ATA TTG CAA AAT CTG ACC CTC 384
Val Thr Thr Arg Asn Glu Met Glu Asn Ile Leu Gln Asn Leu Thr Leu
        115                 120                 125

CGC ATT CAA GTA TCT ATG TTG GGT GCA AAA GGC CAA CCC AGC CCT GGT 432
Arg Ile Gln Val Ser Met Leu Gly Ala Lys Gly Gln Pro Ser Pro Gly
    130                 135                 140

GAG GGA ACA CGA CCA CGA GAA TCA AAC GAC CCC AAC GCC ACC CGA CGT 480
Glu Gly Thr Arg Pro Arg Glu Ser Asn Asp Pro Asn Ala Thr Arg Arg
145                 150                 155                 160

GCC CGC TCC CGC TCC CGG GGA CGT GAA GCA AAG AAA GTG CAA ATT TCT 528
Ala Arg Ser Arg Ser Arg Gly Arg Glu Ala Lys Lys Val Gln Ile Ser
                165                 170                 175

GAT AAG ATC CGT GCC GCA TCC GCT GGG ACC GGG GCC TTG GCA TCA TCA 576
Asp Lys Ile Arg Ala Ala Ser Ala Gly Thr Gly Ala Leu Ala Ser Ser
            180                 185                 190

GCG CCG TCC ACG GCC GTA GCC CAG TCC GCG ACC CCC TCT GTT TCT TCA 624
Ala Pro Ser Thr Ala Val Ala Gln Ser Ala Thr Pro Ser Val Ser Ser
        195                 200                 205

TCT ATT AGC AGC CTC CGG GCC GCG ACT TCG GGG GCG ACT GCC GGC CCC 672
Ser Ile Ser Ser Leu Arg Ala Ala Thr Ser Gly Ala Thr Ala Ala Ala
    210                 215                 220

TCC GCC GCC GCA GCC GTC GAT ACC GGG TCA GGT GGC GGG GGA CAA CCC 720
Ser Ala Ala Ala Ala Val Asp Thr Gly Ser Gly Gly Gly Gly Gln Pro
225                 230                 235                 240

CAC GAC ACC GCC CCA CGC GGG GCA CGT AAG AAA CAG TAG 759
His Asp Thr Ala Pro Arg Gly Ala Arg Lys Lys Gln End
                245                 250

## Patentansprüche

1. Autologes Fusionsprotein, **dadurch gekennzeichnet, daß** es
- eine erste Aminosäuresequenz aufweist, die wenigstens den ersten 100 Aminosäuren, einschließlich des N-Terminus, des p23-Proteins des Epstein-Barr-Virus entspricht und wenigstens
- eine weitere Aminosäuresequenz aufweist, die der Aminosäuresequenz des C-terminalen Bereichs des Epstein-Barr-Virusproteins mit der Bezeichnung p18 oder eines Teils dieses Bereichs entspricht und die immunologischen Eigenschaften eines serologisch (IF) als "Viruskapsid Antigen" definierten späten Antigens aufweist.

2. Autologes Fusionsprotein nach Anspruch 1, **dadurch gekennzeichnet, daß** die erste Aminosäuresequenz wenigstens den ersten 130 Aminosäuren, einschließlich des N-Terminus, des p23-Proteins des Epstein-Barr-Virus entspricht.

3. Autologes Fusionsprotein nach Anspruch 1, **dadurch gekennzeichnet, daß** die erste Aminosäuresequenz der Aminosäuresequenz des p23-Proteins des Epstein-Barr-Virus entspricht.

4. Autologes Fusionsprotein nach einem der vorhergehenden Ansprüche, **dadurch gekennzeichnet, daß** es sich bei der weiteren Aminosäuresequenz um eine Aminosäuresequenz handelt, die bis zu 100 Aminosäuren des C-Terminus des Proteins p18 des Epstein-Barr-Virus entspricht.

5. Autologes Fusionsprotein nach einem der vorhergehenden Ansprüche, **dadurch gekennzeichnet, daß** es die Aminosäuresequenz gemäß Figur 1 aufweist.

6. Testkit zum Nachweis von Antikörpern, insbesondere von IgM- und/oder IgG-Antikörpern gegen das Epstein-Barr-Virus, **dadurch gekennzeichnet, daß** es wenigstens ein Fusionsprotein nach einem der Ansprüche 1 bis 5 umfaßt.

7. Testkit nach Anspruch 6, **dadurch gekennzeichnet, daß** es sich um ein Testkit zur Durchführung eines ELISA-Tests handelt.

8. Verfahren zum Nachweis von Antikörpern, insbesondere von IgM- und/oder IgG-Antikörpern gegen das Epstein-Barr-Virus, **dadurch gekennzeichnet, daß** man eine zu untersuchende Probe mit wenigstens einem Fusionsprotein nach einem der Ansprüche 1 bis 5 zusammenbringt und den Antikörper-Fusionsprotein-Komplex mit einer Nachweiskomponente detektiert.

9. Verfahren nach Anspruch 8, **dadurch gekennzeichnet, daß** die Nachweiskomponente ein Antihuman-Antikörper ist, der gekoppelt ist mit einem Indikatormolekül.

10. Verfahren nach Anspruch 9, **dadurch gekennzeichnet, daß** das Indikatormolekül Meerrettich-Peroxidase ist.

11. Verfahren zum Nachweis von Antikörpern, insbesondere von IgM-Antikörpern gegen das Epstein-Barr-Virus, **dadurch gekennzeichnet, daß** man eine zu untersuchende Probe mit an eine feste Phase gebundenen Antikörpern reagieren läßt, wobei die gebundenen Antikörper gegen die µ-Kette der in der Probe vorhandenen Antikörper gerichtet sind und, daß man die so gebundenen Antikörper mit einem Fusionsprotein nach einem der Ansprüche 1 bis 5 zusammenbringt, wobei das Fusionsprotein eine den Nachweis ermöglichende Markierung aufweist.

## Claims

1. An autologous fusion protein, **characterised in that** it
- comprises a first amino acid sequence which corresponds to at least the first 100 amino acids, including the N-terminus, of the p23 protein of the Epstein-Barr virus and at least
- one further amino acid sequence which corresponds to the amino acid sequence of the C-terminal domain of the Epstein-Barr virus protein designated p18 or a portion of said domain and exhibits the immunological properties of a late antigen defined serologically (IF) as "virus capsid antigen".

2. An autologous fusion protein according to claim 1, **characterised in that** the first amino acid sequence corresponds to at least the first 130 amino acids, including the N-terminus, of the p23 protein of the Epstein-Barr virus.

3. An autologous fusion protein according to claim 1, **characterised in that** the first amino acid sequence corresponds to the amino acid sequence of the p23 protein of the Epstein-Barr virus.

4. An autologous fusion protein according to one of the preceding claims, **characterised in that** the further amino acid sequence comprises an amino acid sequence which corresponds to up to 100 amino acids of the C-terminus of the p18 protein of the Epstein-Barr virus.

5. An autologous fusion protein according to one of the preceding claims, **characterised in that** it comprises the amino acid sequence according to Figure 1.

6. A test kit for the detection of antibodies, in particular of IgM and/or IgG antibodies against the Epstein-Barr virus, **characterised in that** it comprises at least one fusion protein according to one of claims 1 to 5.

7. A test kit according to claim 6, **characterised in that** it is a test kit for performing an ELISA test.

8. A method for the detection of antibodies, in particular of IgM and/or IgG antibodies against the Epstein-Barr virus, **characterised in that** a sample to be investigated is contacted with at least one fusion protein according to one of claims 1 to 5 and the antibody/fusion protein complex is detected with a detection component.

9. A method according to claim 8, **characterised in that** the detection component is an antihuman antibody which is coupled with an indicator molecule.

10. A method according to claim 9, **characterised in that** the indicator molecule is horseradish peroxidase.

11. A method for detecting antibodies, in particular IgM antibodies against the Epstein-Barr virus, **characterised in that** a sample to be investigated is reacted with antibodies bound to a solid phase, wherein the bound antibodies are directed against the µ chain of the antibodies present in the sample and that the antibodies bound in said manner are contacted with a fusion protein according to one of claims 1 to 5, wherein the fusion protein comprises labelling which enables detection.

## Revendications

1. Protéine fusionnée autologue, **caractérisée en ce**
- elle présente une première séquence d'acides aminés correspondant au moins au 100 premiers acides aminés, y compris les extrémités N-terminales, de la protéine p23 du virus d'Epstein-Barr et au moins
- une autre séquence d'acides aminés correspondant à la séquence d'acides aminés de la plage C-terminale du virus d'Epstein-Barr portant la désignation p18, ou à une partie de cette plage, et présentant les propriétés immunologiques d'un antigène tardif sérologiquement (IF) défini comme "antigène de capside du virus".

2. Protéine fusionnée autologue selon la revendication 1, **caractérisée en ce que** la première séquence d'acides aminés correspond à au moins les 130 premiers acides aminés, y compris l'extrémité N-terminale, de la protéine p23 du virus d'Epstein-Barr.

3. Protéine fusionnée autologue selon la revendication 1, **caractérisée en ce que** la première séquence d'acides aminés correspond à la séquence d'acides aminés de la protéine p23 du virus d'Epstein-Barr.

4. Protéine fusionnée autologue selon l'une quelconque des revendications qui précèdent, **caractérisée en ce que** l'autre séquence d'acides aminés est une séquence d'acides aminés qui correspond aux jusqu'à 100 acides aminés de l'extrémité C-terminale de la protéine p18 du virus d'Epstein-Barr.

5. Protéine fusionnée autologue selon l'une quelconque des revendications qui précèdent, **caractérisée en ce qu'**elle présente une séquence d'acides aminés selon la Figure 1.

6. Trousse d'essais pour la détection d'anticorps, en particulier d'anticorps IgM et/ou IgG contre le virus d'Epstein-Barr, **caractérisée en ce qu'**elle contient au moins une protéine fusionnée selon l'une quelconque des revendications 1 à 5.

7. Trousses d'essais selon la revendication 6, **caractérisée en ce qu'**il s'agit d'une trousse d'essais pour l'exécution d'un test ELISA.

8. Procédé pour la détection d'anticorps, en particulier anticorps IgM et/ou IgG contre le virus d'Epstein-Barr, **caractérisé en ce que** l'on met en contact un échantillon à étudier avec au moins une protéine fusionnée selon l'une quelconque des revendications 1 à 5 et on détecte le complexe anticorps - protéine fusionnée à l'aide d'un composant de révélation.

9. Procédé selon la revendication 8, **caractérisé en ce que** le composant révélateur est un anticorps anti-humain, couplé à une molécule d'indicateur.

10. Procédé selon la revendication 9, **caractérisé en ce que** la molécule d'indicateur est de la peroxydase de radis noir.

11. Procédé pour la détection d'anticorps, en particulier anticorps IgM et/ou IgG contre le virus d'Epstein-Barr, **caractérisé en ce que** l'on fait réagir un échantillon à étudier avec des anticorps liés à une phase solide, que les anticorps liés sont dirigés contre la chaîne µ des anticorps présents dans l'échantillon, que l'on met en contact les anticorps ainsi liés avec une protéine fusionnée selon l'une quelconque des revendications 1 à 5, la protéine fusionnée présentant un marqueur qui permet la détection.
